# EUROPÄISCHE PATENTSCHRIFT

(11) **EP 0 292 802 B1**
(45) Veröffentlichungstag und Bekanntmachung des Hinweises auf die Patenterteilung: **06.10.1993**
(21) Anmeldenummer: 88107660.8
(22) Anmeldetag: 13.05.1988
(51) Int. Cl.: C12N 15/10, C07H 21/04

(54) **Verfahren zur Herstellung von doppelsträngiger DNA**
Process for the preparation of double stranded DNA
Procédé de préparation d'ADN double-brin

(30) Priorität: 23.05.1987 DE 3717436
(43) Veröffentlichungstag der Anmeldung: 30.11.1988
(73) Patentinhaber: HOECHST AKTIENGESELLSCHAFT, 65926 Frankfurt am Main (DE)
(72) Erfinder: Uhlmann, Eugen, Dr., D-6246 Glashütten/Taunus (DE); Hein, Friedrich, Dr., D-8000 München 21 (DE)

(56) Entgegenhaltungen:
- EP-A- 0 070 675
- Nucleic Acids Research Symposium Series, Bechyne, 30 August - 5 September 1987, Nr. 18, Seiten 237-240, IRL Press, Oxford, GB, E. Uhlmann et al "Chemoenzymatic synthesis of genes encoding medium-sized polypeptides by use of only one synthetic oligonucleotide"
- Journal of Biological Chemistry, Band 260, Nr. 18, 25. August 1985, Seiten 10271-10277, Am Soc. of Biol. Chemists, Inc. US. K.L. Luskey et al "Human 3-hydroxy-3-methylglutaryl coenzyme A reductase"
- Journal of Biological Chemistry, Band 257, Nr.16, 25 August 1982, Seiten 9226-9229, Am. Soc. of Biol. Chemists, Inc. US. J.J. Rossi et al "An alternate method for synthesis of double-stranded DNA segments"
- Biochemistry, Band 25, 1986, Seiten 4840-4849, Am. Chem. Soc., US, S. Ikuta et al "NMR study of a synthetic DNA hairpin"

## Beschreibung

Die Erfindung betrifft ein leistungsfähiges Verfahren zur Herstellung von DNA-Fragmenten und Genen, das aufgrund der einfachen und rationellen Arbeitsweise auch die Synthese von sehr langen DNA-Sequenzen erlaubt.

Die gängige Methode zur Herstellung doppelsträngiger DNA besteht darin, daß man sich überlappende, einzelsträngige Oligonukleotide chemisch synthetisiert und diese aufgrund der natürlichen Basenpaarung mit Hilfe von DNA-Ligase in die bihelikale Form überführt (H. G. Khorana (1979) Science 203, 614-625). Eine Alternative zur Khorana-Methode ist das sogenannte "fill-in"-Verfahren, bei dem man sich die Reparatur-Eigenschaft der DNA-Polymerase zunutze macht. Hierbei verwendet man zwei chemisch synthetisierte einzelsträngige Oligonukleotide, die an ihrem 3′-Ende einen kurzen Bereich komplementärer Nukleotidsequenzen gemeinsam haben (J. J. Rossi et al. (1982) J. Biol. Chem. 257, 9226-9229). Die erste Methode hat den Nachteil, daß man stets beide Stränge der doppelsträngigen DNA chemisch synthetisieren muß, wobei man zumindest drei Oligonukleotide, im allgemeinen jedoch eine größere Anzahl von Oligonukleotiden, chemisch synthetisieren und enzymatisch verknüpfen muß. Der synthetische Aufwand ist zwar beim "fill-in"-Verfahren im Vergleich zur Khorana-Methode reduziert, man benötigt aber mindestens zwei Oligonukleotide. Ein großes Problem ist ferner das gegenseitige Finden der beiden Oligonukleotide zu gerade der einen definierten Hybridstruktur. Je länger die verwendeten Oligonukleotide sind, desto höher ist die Gefahr der Ausbildung falscher Hybride.

EP-A-0 070 675 beinhaltet eine Methode zur synthese von dsc DNA kodierend für Calcitonin, wobei eine spontane Bildung von Haarnadelstrukturen erfolgt.

Das erfindungsgemäße Verfahren zur Herstellung von doppelsträngiger DNA ist diesen Nachteilen nicht unterworfen. Erfindungsgemäß synthetisiert man einen DNA-Einzelstrang mit einer "Haarnadel"-(hairpin)-Struktur an einem Ende oder an beiden Enden, füllt die komplementären Nukleotide auf und öffnet die Haarnadel-Struktur bzw. -Strukturen oder spaltet sie ab.

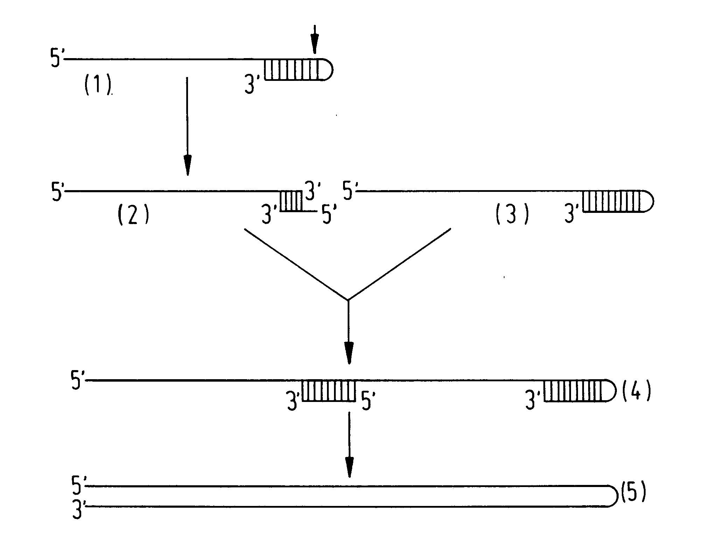

Die Figur erläutert eine spezielle Ausführungsform der Erfindung:
(1) bezeichnet einen DNA-Einzelstrang mit einer Haarnadelstruktur am 3′-Ende, wobei im Bereich der gepaarten Basen (durch senkrechte Striche angedeutet) eine Schnittstelle für ein Restriktionsenzym (durch den kleinen Pfeil angedeutet) liegt.
(2) bezeichnet den nach Schneiden mit dem Restriktionsenzym erhaltenen DNA-Strang, der am 3′-Ende eine kurze Sequenz mit gepaartem Basen (senkrechte Striche) und dem für das Restriktionsenzym charakteristischen "überstehenden" Ende aufweist.
(3) bezeichnet einen anderen DNA-Einzelstrang mit einer Haarnadelstruktur am 3′-Ende, der am 5′-Ende Basen enthält, die komplementär sind zu denen des "überstehenden" Endes von (2).
(4) bezeichnet das Ligationsprodukt von (2) und (3).
(5) bezeichnet den aus (4) erhaltenen aufgefüllten Doppelstrang.

Die Erfindung erlaubt eine Reihe von Ausgestaltungen, die im folgenden näher erläutert werden bzw. in den Patentansprüchen definiert sind. Wenn also im folgenden einzelne Ausführungsformen in der Form vorgestellt werden, daß beispielsweise nur von einer Haarnadel-Struktur die Rede ist, so soll diese Ausdrucksweise nur der Vereinfachung dienen und keinesfalls eine Variation oder Kombination mit anderen Ausführungsformen ausschließen.

In einer bevorzugten Ausgestaltung der Erfindung synthetisiert man zunächst ein Oligonukleotid, das an seinem 3′-Ende eine kurze invertierte Wiederholung in der Sequenz besitzt. Dies führt dazu, daß sich dieses Oligonukleotid an seinem 3′-Ende zurückfaltet und so eine definierte Sekundärstruktur annimmt. Beispielsweise nimmt das Oligonukleotid (1) der Figur die im Anschluß an die Tabelle 1 gezeigte Haarnadelstruktur an. Das so hybridisierte 3′-Ende dient nun als "primer" für die Auffüllung des zweiten DNA-Stranges. Es hat sich gezeigt, daß diese Hybride mit einer Haarnadel-Struktur ungewöhnlich stabil sind und bevorzugt gebildet werden. Die Erfindung erlaubt deshalb die Synthese von praktisch beliebig langen doppelsträngigen DNA-Strukturen, wobei nur ein einziger Oligo- bzw. Polynukleotid-Einzelstrang synthetisiert werden muß. Die Länge des Doppelstrangs ist praktisch nur durch die Synthesemöglichkeiten für den Einzelstrang begrenzt, die laufend weiterentwickelt werden. Für die Synthese des Einzelstrangs kommen alle bekannten Methoden in Betracht, vorteilhaft die Phosphorsäuretriester- bzw. die Phosphitmethode.

Lange einzelsträngige Polynukleotide können gegebenenfalls durch Verknüpfung (Ligation) von Fragmenten hergestellt werden. Die Oligo- bzw. Polynukleotide können sich vom kodierenden oder nichtkodierenden DNA-Strang ableiten und in der 5′-Hydroxy- oder Phosphat-Form eingesetzt werden.

Die Auffüllung zum DNA-Doppelstrang erfolgt nach an sich bekannten Methoden. In einer besonderen Ausgestaltung der Erfindung können jedoch zusätzlich mutagene "Primer" eingesetzt werden, wodurch sich, ausgehend von einem einzigen synthetisierten Oligonukleotid und dem entsprechenden "Primer", beliebige Variationen der DNA-Sequenz erzeugen lassen. Man erhält so, ohne daß eine Klonierung in einzelsträngige Phagen vonnöten wäre, etwa 50 % Mutanten. Nach dieser Methode können beliebige Mutationen - Basenaustausch, Deletion oder Insertion - erzeugt werden. Eine Übersicht über die bekannten Methoden findet sich bei M. Smith (1985), Annu. Rev. Genet. 19, 423-462.

Auch die "misincorporation"-Mutagenese kann angewandt werden (M. Sing et al. (1986) Prot. Engin. 1, 75-76) und ebenso die "saturation"-Mutagenese (K. Derbyshire et al. (1986) Gene 46, 145-152).

Da die Haarnadel-Struktur nicht für eine Verknüpfung mit einem weiteren DNA-Fragment, beispielsweise einem Vektor, geeignet ist, wird diese Struktur entweder geöffnet oder mit Hilfe eines Restriktionsenzyms abgespalten. Geeignete Erkennungsstellen werden in diesem Falle bei der Synthese des Einzelstrangs vorgesehen. Selbstverständlich können auch die bei der Beseitigung der Haarnadel-Struktur entstehenden Enden in an sich bekannter Weise modifiziert werden, also beispielsweise durch Abbau oder Auffüllen stumpfendig gemacht werden oder mit Hilfe von Adaptoren oder Linkern modifiziert werden. Auch in dieser Beziehung erlaubt das erfindungsgemäße Verfahren eine Anpassung an die jeweiligen Erfordernisse. Beispielsweise können auch Erkennungsstellen für Klasse IIS-Restriktionsenzyme, sogenannte "shifters" (W. Szybalski (1985) Gene 40, 169-173), eingebaut werden. Beim Nachschneiden der doppelsträngigen DNA mit diesen Enzymen werden die Erkennungsstellen wieder entfernt, so daß Fragmente an beliebigen Stellen kombiniert werden können. Der Einbau einer oder mehrerer Erkennungsstellen für ein Restriktionsenzym vor der invertierten Wiederholung erlaubt auch eine Restriktionsanalyse zur Feststellung, ob die gewünschte Struktur entstanden ist.

Werden erfindungsgemäß an beiden Enden Haarnadel-Strukturen vorgesehen, so werden zweckmäßig unterschiedliche Erkennungsstellen für Restriktionsenzyme eingebaut, da dann ein Genfragment erzeugt werden kann, das gerichtet in eine Vektorkonstruktion eingesetzt werden kann.

Die Modifizierung der Haarnadel-Struktur kann auch durch Öffnung mit einer einzelstrangspezifischen Nuklease wie "Mung Bean"-Nuklease oder Nuklease S1 erfolgen.

Die Länge der invertierten Wiederholung, die also zum Doppelstrang führt, ist variabel und umfaßt vorteilhaft 8 bis 60, insbesondere 12 bis 32 Basen, entsprechend 4 bis 30, vorteilhaft 6 bis 16 Basenpaarungen. Die Zahl der nicht gepaarten Basen im "loop"-Bereich der Haarnadel-Struktur ist ebenfalls variabel und beträgt vorzugsweise 0 (direkte kovalente Bindung) bis 6.

Konstruiert man eine Haarnadel-Struktur mit günstig gelegener Restriktionsenzym-Schnittstelle (1), so werden nach Abspaltung der Haarnadel-Struktur mit diesem Enzym überstehende Enden (2) erzeugt, die zur Ligation mit einem weiteren Oligonukleotid (3) genutzt werden können. Ein im Ligierungsbereich des erhaltenen langen Polynukleotids (4) eventuell verbleibender kurzer Doppelstrang kann gegebenenfalls durch Hitzedenaturierung entfernt werden, falls in diesem Bereich eine Mutagenese erfolgen soll.

Eine weitere vorteilhafte Ausgestaltung der Erfindung besteht darin, daß bei der Auffüllung zum Doppelstrang eine Markierung erfolgt. In dieser Weise sind besonders vorteilhaft vielfach markierte DNA-Fragmente zugänglich. Auf diese Weise erhaltene hochradioaktiv markierte DNA-Fragmente eignen sich - nach Denaturierung - als Hybridisierungs- bzw. Gensonden.

Es zeigt sich also, daß das erfindungsgemäße Verfahren außerordentlich vielseitig ausgestaltet werden kann und die Synthese sehr langer doppelsträngiger DNA-Fragmente mit einem Minimum an Reaktionsschritten gestattet.

In den folgenden Beispielen wird die Erfindung näher erläutert. Prozentangaben beziehen sich auf das Gewicht, sofern keine anderen Angaben gemacht sind.

### Beispiele

### 1. Chemische Synthese eines einzelsträngigen Oligonukleotids

Am Beispiel des Oligonukleotids 1 (Tabelle 1) wird die Synthese der DNA-Bausteine erläutert. Für die Festphasensynthese wird das am 3′-Ende stehende Nukleosid, im vorliegenden Falle also Thymidin (Nukleotid Nr. 130), über die 3′-Hydroxyfunktion kovalent an einen Träger gebunden verwendet. Trägermaterial ist mit langkettigen Aminoalkylresten funktionalisiertes CPG ("Controlled Pore Glass").

In den folgenden Syntheseschritten wird die Basenkomponente als 5′-O-Dimethoxytritylnukleosid-3′-phosphorigsäure-β-cyanoethylester-dialkylamid eingesetzt, wobei das Adenin als N⁶-Benzoyl-Verbindung, das Cytosin als N⁴-Benzoyl-Verbindung, das Guanin als N²-Isobutyryl-Verbindung und das Thymin ohne Schutzgruppe vorliegen.

25 mg des polymeren Trägers, der 0,2 µmol 5′-O-Dimethoxytrityl-thymidin gebunden enthält, werden nacheinander mit folgenden Agentien behandelt:
A) Acetonitril
B) 3 % Trichloressigsäure in Dichlormethan
C) Acetonitril
D) 5 µmol des entsprechenden Nukleosid-3′-O-phosphits und 25 µmol Tetrazol in 0,15 ml wasserfreiem Acetonitril
E) Acetonitril
F) 20 % Acetanhydrid in Tetrahydrofuran mit 40 % Lutidin und 10 % Dimethylaminopyridin
G) Acetonitril
H) 3 % Jod in Lutidin/Wasser/Tetrahydrofuran im Volumenverhältnis 10:1:40

Unter "Phosphit" wird hierbei der 2′-Desoxyribose-3′-monophosphorigsäure-mono-β-cyanoethylester verstanden, wobei die dritte Valenz durch einen Diisopropylaminorest abgesättigt ist. Die Ausbeuten der einzelnen Syntheseschritte können jeweils nach der Detritylierungsreaktion B) spektrophotometrisch durch Messung der Absorption des Dimethoxytritylkations bei der Wellenlänge von 496 nm bestimmt werden.

Nach abgeschlossener Synthese erfolgt die Abspaltung der Dimethoxytritylgruppe wie in A) bis C) beschrieben. Durch die Behandlung mit Ammoniak wird das Oligonukleotid vom Träger gespalten und zugleich werden die β-Cyanoethylgruppen eliminiert. Eine 16-stündige Behandlung der Oligomeren mit konzentriertem Ammoniak bei 50 °C spaltet die Aminoschutzgruppen der Basen quantitativ ab. Das so erhaltene Rohprodukt wird durch Polyacrylamid-Gelelektrophorese gereinigt.

### 2. Schneiden des Oligonukleotids 1 mit den Restriktionsenzymen EcoRI oder Sau96I

Je 0,015 OD₂₆₀ (o,5 µg) des Oligonukleotids 1 werden in 6 µl Wasser und 2 µl 5x EcoRI-Puffer (50 mM Tris-HCl, pH 7,5; 50 mM Magnesiumchlorid, 250 mM NaCl) gelöst und diese Lösung 5 Minuten auf 95 °C erhitzt. Nach dem Abkühlen auf 37 °C gibt man 2 µl EcoRI (40 units) bzw. 2 µl Sau96I zu und inkubiert 12 Stunden bei 37 °C. Dann werden die Verdauungsansätze auf ein analytisches 10 % Polyacrylamid/7M Harnstoff-Gel aufgetragen und durch Elektrophorese aufgetrennt. Die entstandenen Oligonukleotide laufen gegen einen Marker wie ein 130-mer (Oligonukleotid 1, ungeschnitten), 103-mer (Oligonukleotid 1, EcoRI-geschnitten) und 96-mer (Oligonukleotid 1, Sau96I-geschnitten).

### 3. DNA-Doppelstrang-Bildung und Erzeugung überhängender Enden; Herstellung der DNA-Sequenz I.

0,5 nmol des Oligonukleotids 1 werden in 23 µl Wasser und 5 µl 10x Nick-Translations-Puffer (0,5 M Tris-HCl, pH 7,5; 0,1 M Magnesiumchlorid, 0,1 M DTT, 500 µg/ml BSA) aufgenommen und 5 Minuten auf 95 °C erhitzt. Nachdem die Lösung auf Raumtemperatur abgekühlt ist, gibt man 20 µl dNTP-Lösung (enthaltend je 2,5 mM dATP, TTP, dCTP und dGTP) sowie 2 µl DNA-Polymerase (Klenow-Fragment, 10 units) zu und inkubiert eine Stunde bei Raumtemperatur. Nach 2, 5, 10, 20 und 60 Minuten können 1,5 µl-Proben gezogen werden, um den Verlauf der Reaktion anhand eines 10 %igen nativen Polyacrylamidgels elektrophoretisch zu verfolgen. Als Marker dient pBR322, geschnitten mit MspI. Zur Erzeugung der überhängenden Enden werden 25 µl der DNA-Polymerase-Reaktionslösung 5 Minuten auf 95 °C erhitzt, auf 37 °C temperiert und mit 15 µl 5x EcoRI-Puffer verdünnt. Nach der Zugabe von 12,5 µl 0,2 M NaCl, 37,5 µl Wasser, 5 µl EcoRI und 5 µl HindIII (je 100 units Enzym) inkubiert man 15 Stunden bei 37 °C. Der Ansatz wird durch Elektrophorese auf einem 10 %-igen Polyacrylamidgel (ohne Harnstoff) gereinigt. Die Hauptbande, die gegen den pBR322-Marker wie ein 99 Basenpaarfragment läuft, wird ausgeschnitten und nach Homogenisierung mit 0,2 M Triethylammoniumbikarbonat-Puffer eluiert. Durch Entsalzung über eine ®Sephadex G 50-Säule erhält man das gereinigte DNA-Fragment entsprechend der DNA-Sequenz I (Tabelle 2).

### 4. Herstellung eines mutanten DNA-Fragments (DNA-Sequenz II)

Das für Cystein kodierende Triplett TGT des Oligonukleotids 1 (Nukleotide Nr. 58-60) kann man mit Hilfe des mutagenen Primers

5′ CTTGTCAGTAGAATAGTTAC 3′

in ein für Ser kodierendes Triplett TCT umwandeln.

Hierzu werden zunächst 1 nmol des Primers durch Umsetzung mit Kinase in das entsprechende 5′-Phosphat übergeführt. 0,22 OD₂₆₀ des Primers werden in 15 µl HEPES-Puffer gelöst, diese Lösung 5 Minuten auf 95 °C erhitzt und auf Eis gekühlt. Nach der Zugabe von 9 µl 2,5 mM ATP (pH 7), 2 µl 0,1 M DTT, 3 µl Wasser und 1 µl T4 Polynukleotid-Kinase (10 units) inkubiert man 2 Stunden bei 37 °C und entsalzt anschließend über eine Sephadex G 50-Säule.

Zur Bildung des Doppelstranges werden 0,2 nmol des Oligonukleotids 1 mit 1 nmol des kinasierten Primers wie in Beispiel 3 beschrieben hybridisiert und der DNA-Polymerase-Reaktion unterworfen. Vor dem Nachschneiden mit den Restriktionsenzymen EcoRI und HindIII wird mit Hilfe von DNA-Ligase der neu gebildete DNA-Strang mit dem mutagenen Primer verknüpft. Dazu gibt man 1 µl 10x Ligase-Puffer (660 mM Tris-HCl, pH 7,5; 50 mM Magnesiumchlorid, 50 mM DTT und 10 mM ATP) sowie 1 µl T4 DNA-Ligase zur Polymerase-Reaktion und inkubiert 1,5 Stunden bei 37 °C. Die weitere Bearbeitung erfolgt wie in Beispiel 3 beschrieben. Nach der gelelektrophoretischen Reinigung wird das DNA-Fragment (wie in Beispiel 5 beschrieben) kloniert. Da bei der Mutagenese eine RsaI-Schnittstelle zerstört wird, lassen sich durch Restriktionsanalyse mit RsaI die Mutanten (Ser-kodierend) vom Wildtyp-Plasmid (Cys-kodierend) unterscheiden. Aus dem mutierten Hybridplasmid erhält man durch EcoRI-HindIII-Verdauung ein DNA-Fragment entsprechend der DNA-Sequenz II (Tabelle 3).

### 5. Konstruktion von Vektoren, die die synthetischen DNA-Fragmente enthalten sowie Transformation und Amplifikation derselben

Der handelsübliche Vektor pUC18 wird in bekannter Weise mit den Restriktionsenzymen EcoRI und HindIII nach den Angaben der Hersteller geöffnet. Der Verdauungsansatz wird in bekannter Weise auf einem 1 % Agarosegel durch Elektrophorese aufgetrennt und die Bruchstücke durch Anfärben mit Ethidiumbromid sichtbar gemacht. Die Vektorbande (ca. 2,6 kB) wird aus dem Agarosegel herausgeschnitten und durch Elektroelution, Phenolextraktion und Ethanolfällung von der Agarose getrennt.

10 fmol des Vektors, EcoRI-HindIII-geöffnet, werden mit 100 fmol des synthetischen DNA-Fragments gemäß DNA-Sequenz I bzw. des DNA-Fragments aus Beispiel 4, das die überhängenden Enden entsprechend den Restriktionsenzymen EcoRI und HindIII enthält, in 20 µl Ligase-Puffer in Gegenwart von T4 DNA-Ligase über Nacht bei Raumtemperatur ligiert. Man erhält ein Hybridplasmid, mit dem E. coli transformiert wird. Hierzu wird der Stamm E. coli K12 durch Behandlung mit Calciumchlorid kompetent gemacht und mit der Lösung des Hybridplasmids versetzt. Durch Zugabe von Isopropyl-β-D-thiogalacto-pyranosid (IPTG), 5-Brom-4-chlor-3-indolyl-β-D-galacto-pyranosid (Xgal) und Ampicillin können nach dem Ausplattieren die das Insert im Vektor enthaltenden Kolonien ausfindig gemacht werden. Nach der Amplifikation werden die Zellen aufgeschlossen und die Hybridvektoren nach bekannten Methoden isoliert. Aus diesen lassen sich durch Verdauung mit den ursprünglich benutzten Enzymen EcoRI und HindIII die Genfragmente entsprechend der DNA-Sequenz I bzw. II isolieren. Die Nukleotidsequenzen werden durch die allgemein bekannten Methoden der DNA-Sequenzierung verifiziert.

### 6. Synthese eines Gens für Salm-Calcitonin-Gly(33)

Das Oligonukleotid der DNA-Sequenz III wird wie in Beispiel 1 beschrieben synthetisiert. 0,5 nmol dieses Oligonukleotids werden dann wie in Beispiel 3 beschrieben hybridisiert und mit DNA-Polymerase (Klenow-Fragment) der DNA-Doppelstrang gebildet. Nach der Denaturierung des Enzyms durch Hitzebehandlung wird die NaCl-Konzentration auf 100 mM erhöht und durch Schneiden mit den Restriktionsenzymen SphI und EcoRI (je 100 units) das Gen mit den überstehenden Enden erzeugt.

Die Reinigung und Klonierung des Calcitonin-Genes kann in an sich bekannter Weise erfolgen (beispielsweise wie in der deutschen Patentanmeldung P 36 32 037.4 vorgeschlagen).

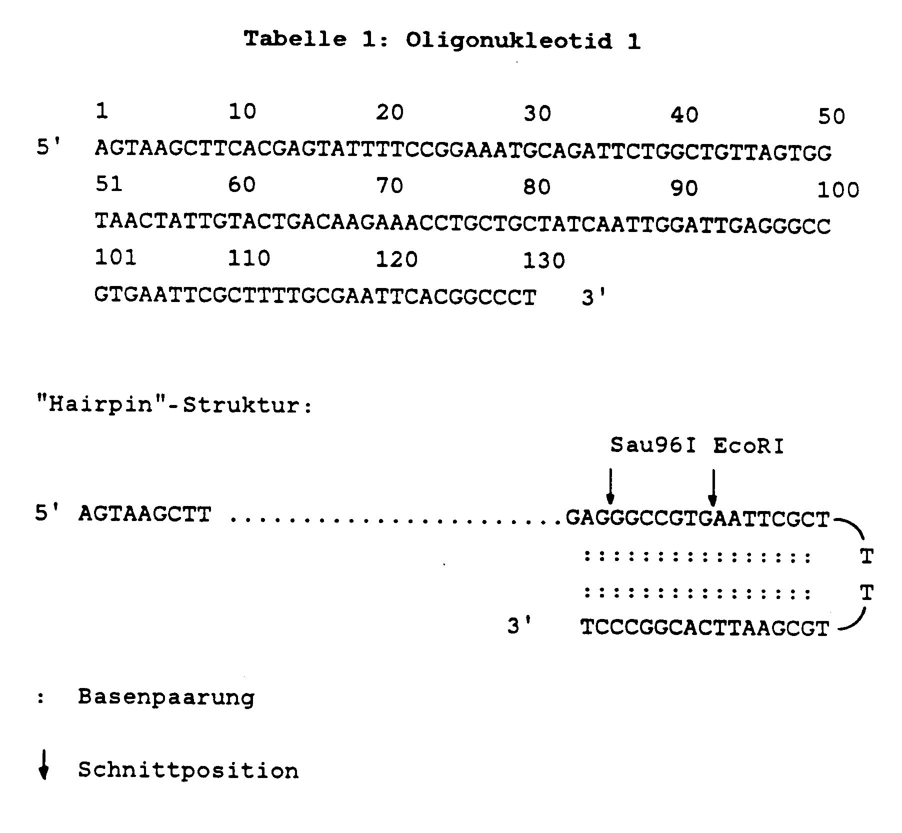
Tabelle 1: Oligonukleotid 1

1         10        20        30        40        50
5' AGTAAGCTTCACGAGTATTTTCCGGAAATGCAGATTCTGGCTGTTAGTGG
51        60        70        80        90        100
TAACTATTGTACTGACAAGAAACCTGCTGCTATCAATTGGATTGAGGGCC
101       110       120       130
GTGAATTCGCTTTTGCGAATTCACGGCCCT   3'

"Hairpin"-Struktur:

Sau96I EcoRI

5' AGTAAGCTT ......................GAGGGCCGTGAATTCGCT
:::::::::::::::: T
:::::::::::::::: T
3' TCCCGGCACTTAAGCGT

: Basenpaarung

 Schnittposition

Tabelle 2: DNA-Sequenz I

LEU HIS GLU TYR PHE PRO GLU MET GLN ILE LEU
AG CTT CAC GAG TAT TTT CCG GAA ATG CAG ATT CTG
A GTG CTC ATA AAA GGC CTT TAC GTC TAA GAC

ALA VAL SER GLY ASN TYR CYS THR ASP LYS LYS PRO
GCT GTT AGT GGT AAC TAT TGT ACT GAC AAG AAA CCT
CGA CAA TCA CCA TTG ATA ACA TGA CTG TTC TTT GGA

ALA ALA ILE ASN TRP ILE GLU GLY ARG
GCT GCT ATC AAT TGG ATT GAG GGC CGT G
CGA CGA TAG TTA ACC TAA CTC CCG GCA CTT AA

Tabelle 3: DNA-Sequenz II

LEU HIS GLU TYR PHE PRO GLU MET GLN ILE LEU
AG CTT CAC GAG TAT TTT CCG GAA ATG CAG ATT CTG
A GTG CTC ATA AAA GGC CTT TAC GTC TAA GAC

ALA VAL SER GLY ASN TYR SER THR ASP LYS LYS PRO
GCT GTT AGT GGT AAC TAT TCT ACT GAC AAG AAA CCT
CGA CAA TCA CCA TTG ATA AGA TGA CTG TTC TTT GGA

ALA ALA ILE ASN TRP ILE GLU GLY ARG
GCT GCT ATC AAT TGG ATT GAG GGC CGT G
CGA CGA TAG TTA ACC TAA CTC CCG GCA CTT AA

Tabelle 4: DNA-Sequenz III

0 1 5 10
Met Cys Ser Asn Leu Ser Thr Cys Val Leu Gly Lys
5' TTTGCATGC ATG TGC TCT AAC CTG TCG ACT TGC GTT CTT GGT AAG
1 10 20 30 40

15 20 25
Leu Ser Gln Glu Leu His Lys Leu Gln Thr Tyr Pro Arg Thr Asn
CTT TCT CAG GAA CTT CAT AAA CTG CAG ACC TAT CCG CGC ACT AAT
50 60 70 80 90

30
Thr Gly Ser Gly Thr Pro Gly Stp Stp
ACC GGC TCT GGT ACC CCT GGT TAA TAG AATTCGCTTTGCGAATTCTATT 3'
100 110 120 130 140

## Patentansprüche (Patentansprüche für folgende(n) Vertragsstaat(en): AT, BE, CH, DE, FR, GB, GR, IT, LI, LU, NL, SE)

1. Verfahren zur Herstellung von doppelsträngiger DNA, dadurch gekennzeichnet, daß man einen DNA-Einzelstrang mit einer Haarnadel-Struktur an einem Ende oder an beiden Enden synthetisiert, die komplementären Nukleotide auffüllt und die Haarnadel-Struktur(en) öffnet oder abspaltet.

2. Verfahren nach Anspruch 1, dadurch gekennzeichnet, daß man benachbart zur Haarnadel-Struktur eine oder mehrere Erkennungsstelle(n) für ein Restriktionsenzym vorsieht, mit dem die Haarnadel-Struktur abgespalten werden kann.

3. Verfahren nach Anspruch 1, dadurch gekennzeichnet, daß man die Haarnadel-Struktur mit einer einzelstrangspezifischen Nuklease öffnet.

4. Verfahren nach einem oder mehreren der vorhergehenden Ansprüche, dadurch gekennzeichnet, daß man die Einzelstrang-DNA mit einem mutagenen "Primer" hybridisiert und eine ortsspezifische Mutagenese durchführt.

5. Verfahren nach einem oder mehreren der vorhergehenden Ansprüche, dadurch gekennzeichnet, daß man beim Auffüllen zum Doppelstrang markierte Nukleotide verwendet.

6. Verfahren nach einem oder mehreren der vorhergehenden Ansprüche, dadurch gekennzeichnet, daß man von dem Oligonukleotid 1 (Tabelle 1) ausgeht.

7. Oligonukleotid 1 (Tabelle 1).

8. DNA-Sequenz III (Tabelle 4).

## Patentansprüche (Patentansprüche für folgende(n) Vertragsstaat(en): ES)

1. Verfahren zur Herstellung von doppelsträngiger DNA, dadurch gekennzeichnet, daß man einen DNA-Einzelstrang mit einer Haarnadel-Struktur an einem Ende oder an beiden Enden synthetisiert, die komplementären Nukleotide auffüllt und die Haarnadel-Struktur(en) öffnet oder abspaltet.

2. Verfahren nach Anspruch 1, dadurch gekennzeichnet, daß man benachbart zur Haarnadel-Struktur eine oder mehrere Erkennungsstelle(n) für ein Restriktionsenzym vorsieht, mit dem die Haarnadel-Struktur abgespalten werden kann.

3. Verfahren nach Anspruch 1, dadurch gekennzeichnet, daß man die Haarnadel-Struktur mit einer einzelstrangspezifischen Nuklease öffnet.

4. Verfahren nach einem oder mehreren der vorhergehenden Ansprüche, dadurch gekennzeichnet, daß man die Einzelstrang-DNA mit einem mutagenen "Primer" hybridisiert und eine ortsspezifische Mutagenese durchführt.

5. Verfahren nach einem oder mehreren der vorhergehenden Ansprüche, dadurch gekennzeichnet, daß man beim Auffüllen zum Doppelstrang markierte Nukleotide verwendet.

6. Verfahren nach einem oder mehreren der vorhergehenden Ansprüche, dadurch gekennzeichnet, daß man von dem Oligonukleotid 1 (Tabelle 1) ausgeht.

## Claims (Claims for the following Contracting State(s): AT, BE, CH, DE, FR, GB, GR, IT, LI, LU, NL, SE)

1. A process for the preparation of double-stranded DNA, which comprises synthesis of a DNA single strand with a hairpin structure at one end or at both ends, filling-in of the complementary nucleotides, and opening or cleavage-off of the hairpin structure(s).

2. The process as claimed in claim 1, wherein there is provision, adjacent to the hairpin structure, of one or more recognition site(s) for a restriction enzyme, using which it is possible to cleave off the hairpin structure.

3. The process as claimed in claim 1, wherein the hairpin structure is opened with a nuclease specific for single strands.

4. The process as claimed in one or more of the preceding claims, wherein the single-stranded DNA is hybridized with a mutagenic primer, and a site-specific mutagenesis is carried out.

5. The process as claimed in one or more of the preceding claims, wherein labeled nucleotides are used when filling in to give the double strand.

6. The process as claimed in one or more of the preceding claims, which starts from the oligonucleotide 1 (Table 1).

7. Oligonucleotide 1 (Table 1).

8. DNA sequence III (Table 4).

## Claims (Claims for the following Contracting State(s): ES)

1. A process for the preparation of double-stranded DNA, which comprises synthesis of a DNA single strand with a hairpin structure at one end or at both ends, filling-in of the complementary nucleotides, and opening or cleavage-off of the hairpin structure(s).

2. The process as claimed in claim 1, wherein there is provision, adjacent to the hairpin structure, of one or more recognition site(s) for a restriction enzyme, using which it is possible to cleave off the hairpin structure.

3. The process as claimed in claim 1, wherein the hairpin structure is opened with a nuclease specific for single strands.

4. The process as claimed in one or more of the preceding claims, wherein the single-stranded DNA is hybridised with a mutagenic primer, and a site-specific mutagenesis is carried out.

5. The process as claimed in one or more of the preceding claims, wherein labeled nucleotides are used when filling in to give the double strand.

6. The process as claimed in one or more of the preceding claims, which starts from the oligonucleotide 1 (Table 1).

## Revendications (Revendications pour l'(les) Etat(s) contractant(s) suivant(s): AT, BE, CH, DE, FR, GB, GR, IT, LI, LU, NL, SE)

1. Procédé pour préparer de l'ADN double brin, caractérisé en ce que l'on effectue la synthèse d'un brin unique d'ADN présentant une structure en épingle à cheveux à une extrémité ou aux deux extrémités, on fournit l'appoint de nucléotides complémentaires et on ouvre ou on scinde la(les) structure(s) en épingle à cheveux.

2. Procédé selon la revendication 1, caractérisé en ce que, à proximité de la structure en épingle à cheveux, on prévoit un ou plusieurs site(s) d'identification pour une enzyme de restriction à l'aide de laquelle on peut scinder la structure en épingle à cheveux.

3. Procédé selon la revendication 1, caractérisé en ce que l'on ouvre la structure en épingle à cheveux à l'aide d'une nucléase spécifique à brin unique.

4. Procédé selon une ou plusieurs des revendications précédentes, caractérisé en ce qu'on hybride l'ADN simple brin avec une "amorce" et on effectue une mutagénèse à localisation spécifique.

5. Procédé selon une ou plusieurs des revendications précédentes, caractérisé en ce qu'on utilise des nucléotides marqués pour la fourniture de l'appoint permettant d'obtenir le double brin.

6. Procédé selon une ou plusieurs des revendications précédentes, caractérisé en ce qu'on prend l'oligonucléotide 1 (tableau 1) pour point de départ.

7. Oligonucléotide 1 (tableau 1).

8. Séquence III d'ADN (tableau 4).

## Revendications (Revendications pour l'(les) Etat(s) contractant(s) suivant(s): ES)

1. Procédé pour préparer de l'ADN à double brin, caractérisé en ce que l'on effectue la synthèse d'un brin unique d'ADN présentant une structure en épingle à cheveux à une extrémité ou aux deux extrémités, on fournit l'appoint de nucléotides complémentaires et on ouvre ou on scinde la(les) structure(s) en épingle à cheveux.

2. Procédé selon la revendication 1, caractérisé en ce que, à proximité de la structure en épingle à cheveux, on prévoit un ou plusieurs site(s) d'identification pour une enzyme de restriction à l'aide de laquelle on peut scinder la structure en épingle à cheveux.

3. Procédé selon la revendication 1, caractérisé en ce que l'on ouvre la structure en épingle à cheveux à l'aide d'une nucléase spécifique à brin unique.

4. Procédé selon une ou plusieurs des revendications précédentes, caractérisé en ce qu'on hybride l'ADN à brin unique avec une "amorce" et on effectue une mutagénèse à localisation spécifique.

5. Procédé selon une ou plusieurs des revendications précédentes, caractérisé en ce qu'on utilise des nucléotides marqués pour la fourniture de l'appoint permettant d'obtenir le double brin.

6. Procédé selon une ou plusieurs des revendications précédentes, caractérisé en ce qu'on prend l'oligonucléotide 1 (tableau 1) pour point de départ.
